(19) Europäisches Patentamt
European Patent Office
Office européen des brevets

(11) EP 0 688 218 B1

(12) **EUROPEAN PATENT SPECIFICATION**

(45) Date of publication and mention of the grant of the patent:
**17.11.1999 Bulletin 1999/46**

(21) Application number: **94907087.4**

(22) Date of filing: **22.02.1994**

(51) Int. Cl.$^6$: **A61K 31/47**

(86) International application number:
**PCT/JP94/00266**

(87) International publication number:
**WO 94/20105 (15.09.1994 Gazette 1994/21)**

(54) **INTERLEUKIN-1 INHIBITOR**
INTERLEUKIN-1 INHIBITOR
INHIBITEUR DE L'INTERLEUKINE-1

(84) Designated Contracting States:
**AT BE CH DE DK ES FR GB GR IT LI LU NL PT SE**

(30) Priority: **10.03.1993 JP 4850193**

(43) Date of publication of application:
**27.12.1995 Bulletin 1995/52**

(73) Proprietor:
**OTSUKA PHARMACEUTICAL CO., LTD.**
**Chiyoda-ku, Tokyo 101-0048 (JP)**

(72) Inventors:
- **ADACHI, Masakazu**
  **Takasaki-shi, Gunma 370 (JP)**
- **TAMAOKA, Hisashi**
  **Itano-gun, Tokushima 771-02 (JP)**
- **ONO, Yukihisa**
  **Itano-gun Tokushima 771-02 (JP)**

(74) Representative:
**von Kreisler, Alek, Dipl.-Chem. et al**
**Patentanwälte,**
**von Kreisler-Selting-Werner,**
**Bahnhofsvorplatz 1 (Deichmannhaus)**
**50667 Köln (DE)**

(56) References cited:
**EP-A- 0 287 951**
**WO-A-91/07401**
**US-A- 4 399 134**
**US-A- 4 894 374**
- **DATABASE WPI Derwent Publications Ltd., London, GB; AN 84-173727 & JP,A,59 095 220 (AJINOMOTO KK) 1 June 1984**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 688 218 B1

## Description

TECHNICAL FIELD

This invention relates to the use of benzoheterocyclic compounds being a 1,4-dihydro-4-oxoquinoline-3-carboxylic acid of the formula:

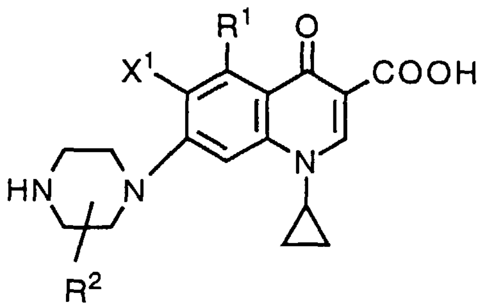

(I)

wherein $R^1$ and $R^2$ are each a $C_{1-6}$ alkyl group and $X^1$ is a halogen atom, or a pharmaceutically acceptable salt thereof, or a 6,7-dihydro-1-oxo-1H,5H-benzo[i,j]quinolidine-2-carboxylic acid of the formula

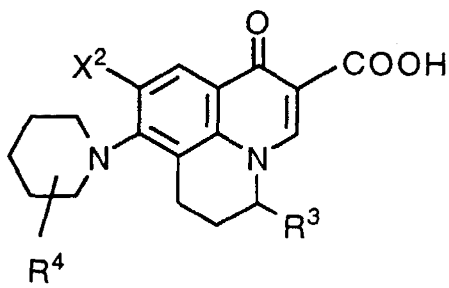

(II)

wherein $R^3$ is a $C_{1-6}$ alkyl group, $R^4$ is a hydroxy group, and $X^2$ is a halogen atom, or a pharmaceutically acceptable salt thereof for preparing a medicament for the prophylaxis and treatment of diseases induced by acceleration of interleukin-1 secretion.

BACKGROUND ART

It has been decided in the 2nd Limphokine Workshop that the physiologically active substance, which had been called by various names such as Lymphocyte Activating Factor (LAF), Mitogenic Protein, Helper peak-1, T-cell replacing factor III (TRF-III), T-cell replacing factor macrophage (TRFM), B-cell activating factor, B-cell differentiation factor, has uniformly been designated as "interleukin-1" (IL-1) [cf. Cellular Immunol., 48, 433-436 (1979)]. This is decided by the reason that the above physiologically active substance having various namings could not be distinguished from each other and had been designated merely based on different angles of the physiological activities.

It has been known that the above IL-1 is a biomaterial which is important for inducing and transmitting the systemic biological response against infection and inflammation, and further this substance per se has a strong antitumor activity [cf. Hirai, Y., et al.; "Gann Monograph on Cancer Research", Japan Scientific Societies Press, Tokyo (1988)], and further it has also been found that it induces response observed in the inflammation in vivo, such as fever, increase of leukocytes, activation of lymphocytes, induction of biosynthesis of acute phase protein in liver [cf. Dinarello, C.A.; Interleukin-1, Rev. Infect. Des., 6, 51-95 (1984), and Kluger, M.J., Oppenheim, J.J. & Powanda, M.C.; The Physiologic, Metabolic and Immunologic Actions of Interleukin-1, Alan R. Liss, Inc., New York (1985)].

Moreover, IL-1 has various biological activities and has been considered to be an important factor for maintaining the homeostasis, but when the function of IL-1 production is disordered and thereby IL-1 is produced in an abnormally larger amount, it may cause various diseases. For example, it has been reported that in case of rheumatoid arthritis, there is a strong correlation between the degree of inflammation of articular synovium and the degree of the bone destruction and expression of HLA-DR antigen in the synovial tissue [cf. Miyasaka, N., Sato, K., Goto, M., Sasano, M., Natsuyama, M., Inoue, K., and Nishioka, K.; Augmented Interleukin-1 Production and HLA-DR Expression in the

Synovium of Rheumatoid Arthritis Patient: Arthritis Rheum., 32, (4), 476-480 (1988)].

Accordingly, it is considered that the various physiological properties associated with IL-1 may be blocked by inhibiting the excess release of IL-1 from cells.

There are used glucocorticoid hormones for the treatment of chronic inflammatory diseases, and it is known that the activities will partly be due to the suppression of IL-1 production [cf. Lew, W., Oppenheim, J.J., & Matsushima, K.; Analysis of the Suppression of IL-1α and IL-1β Production in Human Peripheral Blood Mononuclear Adherent Cells by a Glucocorticoid Hormone: J. Immunol., 140, (6), 1895-1902 (1988)]. However, it has been known that glucocorticoids induce disadvantageously various heavy side effects owing to its various physiological activities.

Thus, it has been desired to find a new drug which has no side effects as observed in glucocorticoids and further has excellent safety in the toxicity and other side effects for the purpose of the treatment of chronic inflammatory diseases such as rheumatoid arthritis, psoriasis, autoimmune diseases.

By the way, the 1,4-dihydro-4-oxoquinoline-3-carboxylic acids of the formula (I) and the 6,7-dihydro-1-oxo-1H,5H-benzo[i,j]quinolidine-2 - carboxylic acids of the formula (II) are disclosed in European Patent Publication 0287951 and U.S. Patent 4,399,134 as antibacterial agents.

Furthermore, U.S. Patent 4,894,374 discloses benzoheterocyclic compounds of the formula

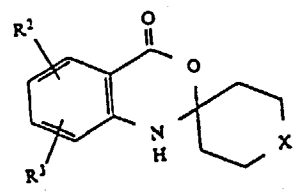

wherein

- X is CHR, NR', S or O;
- R is hydrogen, lower alkyl, lower alkenyl, or unsubstituted or substituted phenyl, naphthyl, pyridyl, quinolinyl, pyrazinyl, pyrimidinyl, quinoxalinyl or quinazolinly, wherein the substituents are selected from halo, carboxy, lower alkoxycarbonyl, lower alkylsulfonyl, cyano, nitro and trifluoromethyl;
- $R^1$ is lower alkenyl, pyridyl, pyrazinyl, pyrimidinyl, quinoxalinyl or quinazolinyl, or substituted phenyl, naphthyl, pyridyl, quinolinyl, pyrazinyl, pyrimidinyl, quinoxalinyl or quinazolinyl, wherein the substituents are selected from halo, carboxy, lower alkoxycarbonyl, lower alkylsulfonyl, cyano, nitro and trifluoromethyl; and
- $R^2$ and $R^3$ are each, independently, hydrogen, halo, lower alkyl, lower alkenyl, lower alkoxy, hydroxy, amino, mono- or dilower-alkylamino, carboxy, lower alkoxycarbonyl, nitro or cyano;

which compounds inhibit interleukin-1 production.

## DISCLOSURE OF THE INVENTION

The present inventors have studied to develop new interleukin-1 inhibitors and have found that the benzoheterocyclic compounds of the formulae (I) and (II) as defined hereinbefore, particularly 7-(3-methyl-1-piperazinyl)-1-cyclopropyl-6-fluoro-5-methyl-1,4-dihydro-4-oxoquinoline-3-carboxylic acid or a salt thereof and 9-fluoro-8-(4-hydroxy-1-piperidinyl)-5-methyl-6,7-dihydro-1-oxo-1H,5H-benzo[i,j]quinolidine-2-carboxylic acid or a salt thereof are useful as an interleukin-1 inhibitor.

Thus, the present invention provides the use of the known benzoheterocyclic compounds of the formulae (I) and (II) and their salts for preparing a medicament for the prophylaxis and treatment of various diseases induced by acceleration of interleukin-1 secretion.

The each group in the formulae (I) and (II) denotes as follows.

The "$C_{1-6}$ alkyl group" denotes a straight chain or branched chain alkyl group having 1 to 6 carbon atoms, such as methyl, ethyl, propyl, isopropyl, butyl, tert-butyl, pentyl and hexyl.

The "halogen atom" denotes fluorine, chlorine, bromine or iodine.

Among the benzoheterocyclic compounds of the formulae (I) and (II), basic compounds can easily form a salt with conventional pharmaceutically acceptable acids. These acids include, for example, inorganic acids such as sulfuric acid, nitric acid, hydrochloric acid, phosphoric acid and hydrobromic acid, and organic acids such as acetic acid, p-toluenesulfonic acid, ethanesulfonic acid, oxalic acid, maleic acid, fumaric acid, malic acid, tartaric acid, citric acid, succinic acid and benzolic acid. Besides, among the benzoheterocyclic compounds of the formulae (I) and (II), acidic compounds can easily form a salt with conventional pharmaceutically acceptable basic compounds. These basic compounds include, for example, sodium hydroxide, potassium hydroxide, calcium hydroxide, sodium carbonate and potassium hydrogen carbonate.

The medicament of the present invention is useful for the prophylaxis and treatment of various diseases induced by acceleration of interleukin-1 secretion, for example, autoimmune diseases such as nephritis, vasculitis, and inflammatory bowel disease (e.g. ulcerative colitis or Crohn's disease); rheumatic diseases such as rheumatoid arthritis, psoriatic arthritis, scleroderma, Behçet's disease, and gout; inflammatory diseases associated with local and systemic infection such as septic shock and inflammatory diseases in dental, ophthalmic and otorhinolic fields such as chronic periodontal disease, ocular inflammatory disease, and otitis media; allergic diseases such as asthma; osteoporosis; endometriosis [cf. Fukih, H. et al; Fertil. Sterl., 47, p213 (1987)]; chronic granulomatous disease; Hodgkin's disease; acute or chronic myelogenous leukemia; graft-vs.-host disease; diabetes [cf. Dayer Metroz M.-D.; Eur. J. Clin. Invest., 22 (No. 4), p2, A50 (1992)]; and Kawasaki's disease [cf. Leung, D.Y.M. et al.; J.Exp.Med., 164, p1958(1986)].

The compounds of the formulae (I) and (II) and their salts can be used in the form of a conventional pharmaceutical preparation in human being and other animals. The medicament is prepared by using conventional diluents or carriers such as fillers, thickening agents, binders, wetting gents, disintegrators, surfactants and lubricants. The medicament may be selected from various forms in accordance with the desired utilities, and the representative forms are tablets, pills, powders, solutions, suspensions, emulsions, granules, capsules, suppositories and injections (solutions, suspensions) . In order to form in tablets, there are used conventional carriers such as vehicles (e.g. lactose, white sugar, sodium chloride, glucose, urea, starches, calcium carbonate, kaolin, crystalline cellulose, silicic acid), binders (e.g. water, ethanol, propanol, simple syrup, glucose solution, starch solution, gelatin solution, carboxymethyl cellulose, shellac, methyl cellulose, potassium phosphate, polyvinylpyrrolidone), disintegrators (e.g. dry starch, sodium arginate, agar powder, laminaran powder, sodium hydrogen carbonate, calcium carbonate, polyoxyethylene sorbitan fatty acid esters, sodium laurylsulfate, stearic monoglyceride, starches, lactose), disintegration inhibitors (e.g. white sugar, stearin, cacao butter, hydrogenated oils), absorption promoters (e.g. quaternary ammonium base, sodium laurylsulfate), wetting agents (e.g. glycerin, starches), adsorbents (e.g. starches, lactose, kaolin, bentonite, colloidal silicates) and lubricants (e.g. purified talc, stearates, boric acid powder, polyethylene glycol). Moreover, the tablets may also be in the form of a conventional coated tablet, such as sugar-coated tablets, gelatin-coated tablets, enteric coated tablets, film coating tablets, or double or multiple layer tablets.

In the preparation of pills, the carriers include vehicles (e.g. glucose, lactose, starches, cacao butter, hydrogenated vegetable oils, kaolin, talc), binders (e.g. gum arabic powder, tragacanth powder, gelatin, ethanol) and disintegrators (e.g. laminaran, agar). In the preparation of suppositories, the carriers include, for example, polyethylene glycol, cacao butter, higher alcohols, higher alcohol esters, gelatin and semi-synthetic glycerides.

Capsules can be prepared by charging a mixture of the compound of this invention with the above carriers into hard gelatin capsules or soft capsules in a usual manner.

In the preparation of injections, the solutions, emulsions or suspensions are sterilized and are preferably made isotonic with the blood. In the preparation of these solutions, emulsions and suspensions, there are used conventional diluents, such as water, macrogol, ethyl alcohol, propylene glycol, ethoxylated isostearyl alcohol, polyoxylated isostearyl alcohol and polyoxyethylene sorbitan fatty acid esters. In this case, the pharmaceutical preparations may also be incorporated with sodium chloride, glucose or glycerin in an amount sufficient to make them isotonic, and may also be incorporated with conventional solubilizers, buffers, anesthetizing agents.

Besides, the medicament may optionally be incorporated with coloring agents, preservatives, perfumes, flavors, sweetening agents, and other medicaments, if desired.

The amount of the active compound to be incorporated into the medicament of this invention is not specified but may be selected from a broad range, but it is usually in the range of 1 to 70% by weight, preferably 1 to 30% by weight.

The medicament of this invention may be administered in any method, and suitable method for administration may be determined in accordance with various forms of preparation, ages, sexes and other conditions of the patient, the degree of severity of diseases. For example, tablets, pills, solutions, suspensions, emulsion, granules and capsules are administered orally. The injections are intravenously administered alone or together with a conventional auxiliary liquid (e.g. glucose, amino acid solutions), and further are optionally administered alone in intramuscular, intracutaneous, subcutaneous, or intraperitoneal route, if desired. Suppositories are administered in intrarectal route.

The dosage of the medicament of this invention may be selected in accordance with the usage, ages, sexes and other conditions of the patient, the degree of severity of the diseases, but is usually in the range of 0.1 to 1000 mg of the active compound per 1 kg of body weight of the patient per day. The daily dosage may be administered dividedly in one to four times in a day. The active compound is preferably contained in an amount of 1 to 600 mg per the dosage unit.

BEST MODE FOR CARRYING OUT THE INVENTION

The present invention is illustrated by the following preparations and pharmacological experiments.

Preparation 1

Film coated tablets are prepared from the following components.

| Components | Amount |
|---|---|
| 7-(3-Methyl-1-piperazinyl)-1-cyclopropyl-6-fluoro-5-methyl-1,4-hydroxy-4-oxoquinoline-3-carboxylic acid | 150 g |
| Avicel ®(tradename of microcrystalline cellulose, manufactured by Asahi Chemical Industry Co., Ltd., Japan) | 40 g |
| Corn starch | 30 g |
| Magnesium stearate | 2 g |
| Hydroxypropyl methylcellulose | 10 g |
| Polyethylene glycol-6000 | 3 g |
| Castor oil | 40 g |
| Ethanol | 40 g |

The active component, Avicel®, corn starch and magnesium stearate are mixed and kneaded and the mixture is tabletted using a conventional pounder (R 10 mm) for sugar coating. The tablets thus obtained are coated with a film coating agent consisting of hydroxypropyl methylcellulose, polyethylene glycol-6000, castor oil and ethanol to give film coated tablets.

Preparation 2

Tablets are prepared from the following components.

| Components | Amount |
|---|---|
| 9-Fluoro-8-(4-hydroxy-1-piperidinyl)-5-methyl-6,7-dihydro-1-oxo-1H,5H-benzo[ji]quinolidine-2-carboxylic acid | 150 g |
| Citric acid | 1.0 g |
| Lactose | 33.5 g |
| Dicalcium phosphate | 70.0 g |
| Pluronic®F-68 | 30.0 g |
| Sodium laurylsulfate | 15.0 g |
| Polyvinylpyrrolidone | 15.0 g |
| Polyethylene glycol (Carbowax®1500) | 4.5 g |
| Polyethylene glycol (Carbowax® 6000) | 45.0 g |
| Corn starch | 30.0 g |
| Dry sodium laurylsulfate | 3.0 g |
| Dry magnesium stearate | 3.0 g |

(continued)

| Components | Amount |
|---|---|
| Ethanol | q.s. |

The active compound of this invention, citric acid, lactose, dicalcium phosphate, Pluronic® F-68 and sodium laurylsulfate are mixed. The mixture is screened with No. 60 screen and is granulated in wet with an alcohol solution containing polyvinylpyrrolidone, carbowax® 1500 and 6000. If required, an alcohol is added thereto so that the powder mixture is made a paste-like mass. Corn starch is added to the mixture and the mixture is continuously mixed to form uniform particles. The resulting particles are passed through No. 10 screen and entered into a tray and then dried in an oven at 100°C for 12 to 14 hours. The dried particles are screened with No. 16 screen and thereto are added dry sodium laurylsulfate and dry magnesium stearate, and the mixture is tabletted to form the desired shape.

The core tablets thus prepared are varnished and dusted with talc in order to guard from wetting. Undercoating is applied to the core tablets. In order to administer the tablets orally, the core tablets are vanished several times. In order to give round shape and smooth surface to the tablets, further under - coating and coating with lubricant are applied thereto. The tablets are further coated with a coloring coating material until the desired colored tablets are obtained. After drying, the coated tablets are polished to obtain the desired tablets having uniform gloss.

Pharmacological experiment

Method: A 10% heparinized prepheral blood from healthy volunteer, a test compound and lipopolysaccharide (LPS, 3.3 μg/ml) were suspended in RPMI-1640 medium supplemented with penicillin 100 units/ml and streptomycin 0.1 μg/ml, and the mixture was incubated in a 5% $CO_2$ atmosphere at 37°C for 18 - 24 hours. The supernatant of the culture was collected by centrifugation.

The IL-1α and IL-1β isolated from cells by stimulation with LPS were measured by enzyme-linked immunoassay (ELISA). That is, 96-well ELISA plate was coated with a mouse monoclonal antibody against human IL - 1α or human IL-1β, followed by blocking treatment, and thereto was added a test sample and it was subjected to reaction. After the reaction, the plate was washed, and then rabbit polyclonal antibody against IL-1α or IL-1β was added to the plate and subjected to reaction. After washing the plate, horseradish peroxidase (POD)-conjugated anti-rabbit immunoglobulin was added thereto and subjected to reaction. After removing the unbound POD-conjugated antibody by washing, a substrate solution (containing ortho-phenylenediamine and hydrogen peroxide) was added and subjected to reaction, and thereafter, the absorbance at 492 nm was measured, and thereby the amounts of IL-1α and IL-1β were measured based on each standard curve. The ratio (%) of inhibition of IL-1 release was calculated by the following equation:

$$\text{IL-1 release inhibitory ratio (\%)} = 100 \times (1-T/C)$$

wherein T means the amount of IL-1 in the supernatant of culture incorporated with the test compound, and C means the amount of IL-1 in the supernatant of culture added only with the solvent.

Test compounds:

1. 7-(3-Methyl-1-piperazinyl)-1-cyclopropyl-6-fluoro-5-methyl-1,4-dihydro-4-oxoquinoline-3-carboxylic acid.
2. 9-Fluoro-8-(4-hydroxy-1-piperidinyl)-5-methyl-6,7-dihydro-1-oxo-1H,5H-benzo[ji]quinolidine-2-carboxylic acid.

Results:

The results are shown in Table 1.

Table 1

| Test compounds | Dose ($3 \times 10^{-5}$ g/ml) | |
|---|---|---|
| | IL-1α release inhibitory ratio | IL-1β release inhibitory ratio |
| 1 | 93 % | 96 % |
| 2 | 82 % | 78 % |

INDUSTRIAL APPLICATION

The medicament of this invention is useful for the prophylaxis and treatment of various diseases induced by acceleration of IL-1 secretion, such as autoimmune diseases, rheumatic diseases, various inflammatory diseases and allergic diseases in human being and animals.

## Claims

1. Use of a benzoheterocyclic compound of the formula

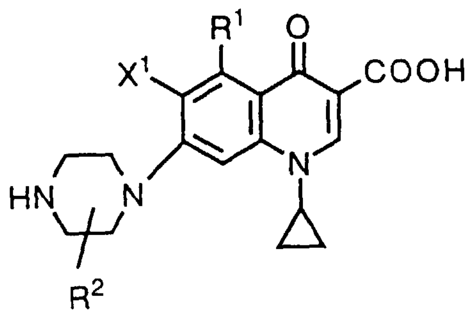

(I)

wherein $R^1$ and $R^2$ are each a $C_{1-6}$ alkyl group and $X^1$ is a halogen atom, or

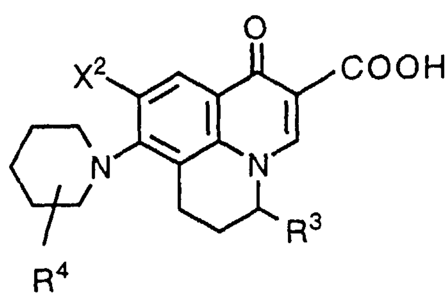

(II)

wherein $R^3$ is a $C_{1-6}$ alkyl group, $R^4$ is a hydroxy group, and $X^2$ is a halogen atom, or a pharmaceutically acceptable salt thereof for preparing a medicament for the prophylaxis and treatment of diseases induced by acceleration of interleukin-1 secretion.

2. The use according to claim 1, wherein the diseases induced by acceleration of interleukin-1 secretion is/are autoimmune diseases, rheumatic diseases, inflammatory diseases associated with local and systemic infection, allergic diseases, osteoporosis, endometriosis, chronic granulomatous disease, Hodgkin's disease, acute or chronic myelogenous leukemia, graft-vs.-host disease, diabetes, and Kawasaki's disease.

3. The use according to claim 2, wherein the diseases induced by acceleration of interleukin-1 secretion are autoimmune diseases and rheumatic diseases.

4. The use according to claim 1, 2 or 3, wherein the active compound is 7-(3-methyl-1-piperazinyl)-1-cyclopropyl-6-fluoro-5-methyl-1,4-dihydro-4-oxoquinoline-3-carboxylic acid or 9-fluoro-8-(4-hydroxy-1-piperidinyl)-5-methyl-6,7-dihydro-1-oxo-1H,5H-benzo[ij]quinolidine-2-carboxylic acid, or a pharmaceutically acceptable salt thereof.

## Patentansprüche

1. Verwendung einer benzoheterocyclischen Verbindung der Formel

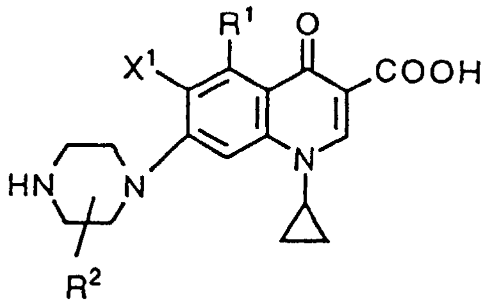

(I) ,

wobei $R^1$ und $R^2$ jeweils eine $C_{1-6}$-Alkylgruppe sind und $X^1$ ein Halogenatom ist, oder

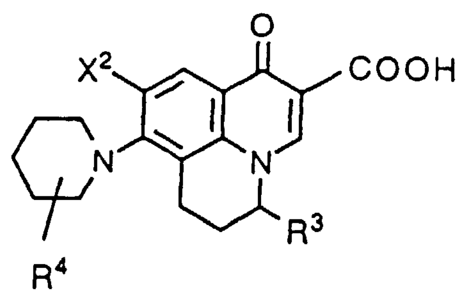

(II) ,

wobei $R^3$ eine $C_{1-6}$-Alkylgruppe ist, $R^4$ eine Hydroxygruppe ist und $X^2$ ein Halogenatom ist, oder eines pharmazeutisch verträglichen Salzes davon zur Herstellung eines Medikaments zur Prophylaxe und Behandlung von Krankheiten, die durch eine Beschleunigung der Sekretion von Interleukin 1 induziert werden.

2. Verwendung nach Anspruch 1, wobei die durch die Beschleunigung der Sekretion von Interleukin 1 induzierten Krankheiten Autoimmun-Krankheiten, rheumatische Erkrankungen, entzündliche Erkrankungen in Verbindung mit einer lokalen und systemischen Infektion, allergische Erkrankungen, Osteoporose, Endometriose, eine chronische granulomatöse Erkrankung, die Hodgkinsche Krankheit, akute oder chronische myelogene Leukämie, eine Transplantat-gegen-Empfänger-Reaktion, Diabetes und das Kawasaki-Syndrom ist/sind.

3. Verwendung nach Anspruch 2, wobei die durch die Beschleunigung der Sekretion von Interleukin 1 induzierten Krankheiten Autoimmun-Krankheiten und rheumatische Erkrankungen sind.

4. Verwendung nach Anspruch 1, 2 oder 3, wobei die aktive Verbindung 7-(3-Methyl-1-piperazinyl)-1-cyclopropyl-6-fluor-5-methyl-1,4-dihydro-4-oxochinolin-3-carbonsäure oder 9-Fluor-8-(4-hydroxy-1-piperidinyl)-5-methyl-6,7-dihydro-1-oxo-1H,5H-benzo[ij]chinolidin-2-carbonsäure oder ein pharmazeutisch annehmbares Salz davon ist.

## Revendications

1. Utilisation d'un composé benzohétérocyclique de formule

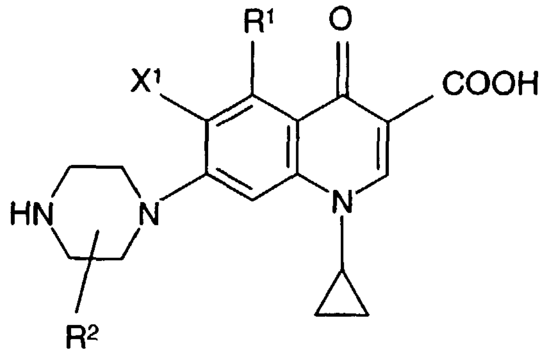

(I)

où $R^1$ et $R^2$ sont chacun un groupe alkyle en $C_{1-6}$ et $X^1$ est un atome d'halogène, ou

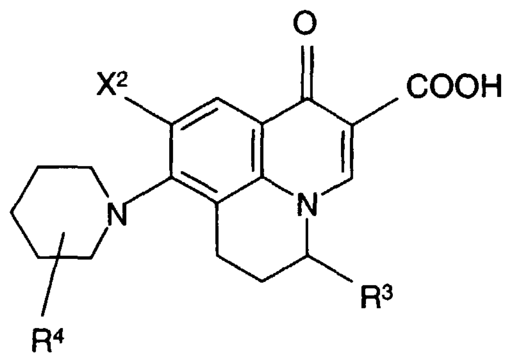

(II)

où $R^3$ est un groupe alkyle en $C_{1-6}$, $R^4$ est un groupe hydroxyle et $X^2$ est un atome d'halogène, ou d'un sel pharmaceutiquement acceptable de celui-ci, pour préparer un médicament pour la prophylaxie et le traitement des maladies provoquées par une accélération de la sécrétion d'interleukine 1.

2. Utilisation selon la revendication 1 dans laquelle les maladies provoquées par une accélération de la sécrétion d'interleukine 1 sont des maladies auto-immunes, des maladies rhumatismales, des maladies inflammatoires associées à une infection locale et systémique, des maladies allergiques, l'ostéoporose, l'endométriose, la granulomatose chronique, la maladie de Hodgkin, la leucémie myéloïde aiguë ou chronique, la maladie du greffon contre l'hôte, le diabète et la maladie de Kawasaki.

3. Utilisation selon la revendication 2 dans laquelle les maladies provoquées par une accélération de la sécrétion d'interleukine 1 sont des maladies auto-immunes et des maladies rhumatismales.

4. Utilisation selon la revendication 1, 2 ou 3 dans laquelle le composé actif est l'acide 7-(3-méthyl-1-pipérazinyl)-1-cyclopropyl-6-fluoro-5-méthyl-1, 4-dihydro-4-oxoquinoléine-3-carboxylique ou l'acide 9-fluoro-8-(4-hydroxy-1-pipéridinyl)-5-méthyl-6,7-dihydro-1-oxo-1H,5H-benzo[i,j]quinolidine-2-carboxylique, ou un sel pharmaceutiquement acceptable de celui-ci.